# EUROPEAN PATENT SPECIFICATION

(11) **EP 3 094 722 B1**
(45) Date of publication and mention of the grant of the patent: **12.05.2021**
(21) Application number: 14700478.2
(22) Date of filing: 14.01.2014
(51) Int. Cl.: C12N 9/02, C12N 15/82

(54) **PHYTOPHTHORA RESISTANT PLANTS BELONGING TO THE SOLANACEAE FAMILY**
PHYTOPHTHORA-RESISTENTE PFLANZEN DER FAMILIE SOLANACEAE
PLANTES RÉSISTANTES À PHYTOPHTHORA APPARTENANT À LA FAMILLE DES SOLANACÉES

(43) Date of publication of application: 23.11.2016
(73) Proprietor: Enza Zaden Beheer B.V., 1602 DB Enkhuizen (NL)
(72) Inventor: VAN SCHIE, Christianus Cornelis Nicolaas, NL-1087 LL Amsterdam (NL); POSTHUMA, Karin Ingeborg, NL-1601 AJ Enkhuizen (NL); ZEILMAKER, Tieme, NL-3823 JJ Amersfoort (NL); KROON, Maria Theresia, NL-2134 PP Hoofddorp (NL); DE BOER, Geert Johannes, NL-1974 LV IJmuiden (NL)
(74) Representative: van Kooij, Adriaan
(86) International application number: PCT/EP2014/050572
(87) International publication number: WO 2015/106796

(56) References cited:
- EP-A1- 2 455 473
- WO-A1-2008/092505
- WO-A1-2008/092659
- Tieme Zeilmaker: "Functional and applied aspects of the DOWNY MILDEW RESISTANT 1 and 6 genes in Arabidopsis (PhD thesis)", , 6 February 2012 (2012-02-06), pages FP-147, XP002730064, Universiteit Utrecht Retrieved from the Internet: URL:http://web.science.uu.nl/pmi/publicati ons/PDF/2012/Proefschrift-Zeilmaker-2012.p df [retrieved on 2014-09-23]
- DATABASE UniProt [Online] 3 April 2013 (2013-04-03), "SubName: Full=Uncharacterized protein;", XP002730065, retrieved from EBI accession no. UNIPROT:M0ZIQ1 Database accession no. M0ZIQ1 & XU XUN ET AL: "Genome sequence and analysis of the tuber crop potato", NATURE (LONDON), vol. 475, no. 7355, July 2011 (2011-07), page 189, ISSN: 0028-0836(print)
- DATABASE UniProt [Online] 3 April 2013 (2013-04-03), "SubName: Full=Uncharacterized protein;", XP002730066, retrieved from EBI accession no. UNIPROT:M1CK41 Database accession no. M1CK41 & XU XUN ET AL: "Genome sequence and analysis of the tuber crop potato", NATURE (LONDON), vol. 475, no. 7355, July 2011 (2011-07), page 189, ISSN: 0028-0836(print)
- DATABASE UniProt [Online] 28 November 2012 (2012-11-28), "SubName: Full=Uncharacterized protein;", XP002730067, retrieved from EBI accession no. UNIPROT:K4C928 Database accession no. K4C928 & BUDIMAN MUHAMMAD A ET AL: "A deep-coverage tomato BAC library and prospects toward development of an STC framework for genome sequencing", GENOME RESEARCH, vol. 10, no. 1, January 2000 (2000-01), pages 129-136, ISSN: 1088-9051

## Description

The present invention relates to *Phytophthora* resistance plants belonging to *Solanaceae* family wherein said resistance is encoded by a combination of two genes.

The plant pathogen *Phytophthora* is a genus of plant-damaging Oomycetes (water molds), whose member species are capable of causing large economic losses on crops worldwide, as well as environmental damage in natural ecosystems. The genus was first described by Heinrich Anton de Bary in 1875. Approximately 100 species have been described, although and estimate of 100 to 500 undiscovered *Phytophthora* species are suspected to exist.

*Phytophthora* pathogens are mostly pathogens of dicotyledons and generally are host-specific parasites. Many species of *Phytophthora* are plant pathogens of considerable economic importance. *Phytophthora infestans* was the infective agent of the potato blight that caused the Great Irish Famine (1845-1849), and still remains the most destructive pathogen of solanaceous crops, including tomato and potato. The soya bean root and stem rot agent, *Phytophthora sojae,* has also caused longstanding problems for the agricultural industry. In general, plant diseases caused by this genus are difficult to control chemically, and thus the growth of resistant cultivars is the main management strategy. Other important Phytophthora diseases are: *Phytophthora cactorum* - causes rhododendron root rot affecting rhododendrons, azaleas and causes bleeding canker in hardwood trees; *Phytophthora capsici* - infects Cucurbitaceae fruits, such as cucumbers and squash, *Phytophthora cinnamomi* - causes cinnamon root rot affecting woody ornamentals including arborvitae, azalea, *Phytophthora fragariae* - causes red root rot affecting strawberries; *Phytophthora kernoviae* - pathogen of beech and rhododendron, also occurring on other trees and shrubs including oak, and holm oak, *Phytophthora megakarya* - one of the cocoa black pod disease species, is invasive and probably responsible for the greatest cocoa crop loss in Africa; *Phytophthora palmivora* - causes fruit rot in coconuts and betel nuts, *Phytophthora ramorum*, *Phytophthora quercina* - causes oak death, and *Phytophthora sojae -* causes soybean root rot.

*Phytophthora* is sometimes referred to as a fungal-like organism but it is classified under a different kingdom: *Chromalveolata* (formerly *Stramenopila* and previously *Chromista*). *Phytophthora* is morphologically very similar to true fungi yet its evolutionary history is quite distinct. In contrast to fungi, chromalveolatas are more closely related to plants than animals. Whereas fungal cell walls are made primarily of chitin, chromalveolata cell walls are constructed mostly of cellulose. Ploidy levels are different between these two groups; *Phytophthora* have diploid (paired) chromosomes in the vegetative (growing, non-reproductive) stage of life, Fungi are almost always haploid in this state. Biochemical pathways also differ, notably the highly conserved.

*Phytophthoras* may reproduce sexually or asexually. In many species, sexual structures have never been observed, or have only been observed in laboratory matings. In homothallic species, sexual structures occur in single culture. Heterothallic species have mating strains, designated as A1 and A2. When mated, antheridia introduce gametes into oogonia, either by the oogonium passing through the antheridium (amphigyny) or by the antheridium attaching to the proximal (lower) half of the oogonium (paragyny), and the union producing oospores. Like animals, but not like most true fungi, meiosis is gametic, and somatic nuclei are diploid. Asexual (mitotic) spore types are chlamydospores, and sporangia which produce zoospores. Chlamydospores are usually spherical and pigmented, and may have a thickened cell wall to aid in its role as a survival structure. Sporangia may be retained by the subtending hyphae (non-caducous) or be shed readily by wind or water tension (caducous) acting as dispersal structures. Also, sporangia may release zoospores, which have two unlike flagella which they use to swim towards a host plant.

*The Solanaceae,* or nightshades, are an economically important family of flowering plants. The family ranges from herbs to trees, and includes a number of important agricultural crops, medicinal plants, spices, weeds, and ornamentals. Many members of the family contain potent alkaloids, and some are highly toxic.

The family belongs to the order *Solanales,* in the asterid group dicotyledons (*Magnoliopsida*). The solanaceae family consists of approximately 98 genera and some 2,700 species, with a great diversity of habitats, morphology and ecology.

The family has a worldwide distribution being present on all continents except Antarctica. The greatest diversity in species is found in South America and Central America. *Solanaceae* includes a number of commonly collected or cultivated species. Perhaps the most economically important genus of the family is *Solanum,* which contains the potato (*Solanum tuberosum,* in fact, another common name of the family is the "potato family"), the tomato (*Solanum lycopersicum*), and the aubergine or eggplant (*Solanum melongena*). Another important genus *Capsicum* produce both chilli peppers and bell peppers.

The genus *Physalis* produces the so-called groundcherries, as well as the tomatillo (*Physalis philadelphica*), the Cape gooseberry and the Chinese lantern. The genus *Lycium* contains the boxthorns and the wolfberry *Lycium barbarum. Nicotiana* contains, among other species, the plant that produces tobacco. Some other important members of *Solanaceae* include a number of ornamental plants such as Petunia, Browallia and Lycianthes, the source of psychoactive alkaloids, Datura, Mandragora (mandrake), and Atropa belladonna (deadly nightshade). Certain species are universally known for their medicinal uses, their psychotropic effects or for being poisonous.

With the exception of tobacco (*Nicotianoideae*) and petunia (*Petunioideae*), most of the economically important genera are contained in the subfamily *Solanoideae.* Finally, but not less importantly, the solanaceas include many model organisms which are important in the investigation of fundamental biological questions at a cellular, molecular and genetic level, such as tobacco and the petunia.

WO 2008/092505 discloses plants being resistant to a pathogen of viral, bacterial, fungal or oomycete origin, wherein the plants have a reduced level, reduced activity or complete absence of DMR6 protein as compared to a plant that is not resistant to the pathogen, in particular organisms of the Fungi or the phylum Oomycota.

Considering the economic importance of many plant members of the *Solanaceae* family and the destructive effect of the plant pathogen *Phytophthora* on many members of this family, it is an object, amongst other objects, of the present invention to provide *Phytophthora* resistant plants.

The above object, amongst other objects, is met by the present invention by providing plants as outlined in the appended claims.

Specifically, the above object, amongst other objects, is met, according to a first aspect, by plants belonging to the *Solanaceae* family wherein the present plants comprise a genetic trait providing *Phytophthora* resistance and wherein the present resistance trait is encoded by a combination of at least two genes having a reduced expression, or reduced transcription, of the present genes or a reduced activity of proteins encoded by the present genes as compared to the plant belonging to *Solanaceae* family being susceptible to *Phytophthora.* According to the present invention, the present plants belonging to the *Solanaceae* family are selected from the group consisting of potato, petunia and tomato.

The present invention relates to potato, the present *Phytophthora* resistance is resistance to *Phytophthora infestans* and the present combination of at least two genes are genes encoding proteins according to SEQ ID No. 1 and SEQ ID No. 2 or proteins having at least 90% sequence identity with SEQ ID No. 1 and SEQ ID No. 2, such as 91%, 92%, 93% and 94% sequence identity, preferably at least 95% sequence identity, such as 96%, 97%, 98% and 99% sequence identity.

The present invention relates to petunia, the present *Phytophthora* resistance is resistance to *Phytophthora nicotianae* and the present combination of at least two genes are genes encoding proteins according to SEQ ID No. 3 and SEQ ID No. 4 or proteins having at least 90% sequence identity with SEQ ID No. 3 and SEQ ID No. 4, such as 91%, 92%, 93% and 94% sequence identity, preferably at least 95% sequence identity, such as 96%, 97%, 98% and 99% sequence identity.

The present invention relates to tomato, the present *Phytophthora* resistance is resistance to *Phytophthora infestans* and the present combination of at least two genes are genes encoding proteins according to SEQ ID No. 5 and SEQ ID No. 6 or proteins having at least 90% sequence identity with SEQ ID No. 5 and SEQ ID No. 6, such as 91%, 92%, 93% and 94% sequence identity, preferably at least 95% sequence identity, such as 96%, 97%, 98% and 99% sequence identity.

The present invention relates to a plant belonging to the *Solanaceae* family wherein the present plant comprises a genetic trait providing *Phytophthora* resistance, wherein the present resistance trait is obtained by down regulating the activity of combination of two genes or reducing the activity of proteins encoded by the present genes in a *Phytophthora* susceptible plant, wherein the present two genes encode the combinations of SEQ ID Nos. 1 and 2 or SEQ ID Nos. 3 and 4 or SEQ ID Nos. 5 and 6 or proteins having at least 90% sequence identity therewith such as 91%, 92%, 93% and 94% sequence identity, preferably at least 95% sequence identity, such as 96%, 97%, 98% and 99% sequence identity.

Given the advantageous properties of the disclosed genes for providing *Phytophthora* resistance plants, discloses is the use of genes encoding the combinations of SEQ ID Nos. 1 and 2 or SEQ ID Nos. 3 and 4 or SEQ ID Nos. 5 and 6 or proteins having at least 90% sequence identity therewith, such as 91%, 92%, 93% and 94% sequence identity, preferably at least 95% sequence identity, such as 96%, 97%, 98% and 99% sequence identity, for providing *Phytophthora* resistance in plants belonging to the *Solanaceae* family.

The disclosed use for providing *Phytophthora* resistance in plants belonging to the *Solanaceae* family comprises reduced expression of the present genes or a reduced activity of proteins encoded by the present genes as compared to the plant belonging to *Solanaceae* family being susceptible to *Phytophthora.*

The present plants belonging to the *Solanaceae* family are selected from the group consisting of potato, petunia and tomato. The present *Phytophthora* resistance is *Phytophthora infestans* in potato and/or tomato, or *Phytophthora nicotianae* in petunia.

Given the *Phytophthora* resistance providing properties of the present proteins and genes, disclosed are proteins and genes suitable for providing *Phytophthora* resistance to plants. Specifically disclosed are proteins selected from the group consisting of SEQ ID No. 1, 2, 3, 4, 5, 6.

Disclosed are coding sequences, or genes encoding cDNA sequence, selected from the group consisting of SEQ ID No. 7, 8, 9, 10, 11, 12.

The invention is further illustrated in the examples below, with reference to the figures, wherein:

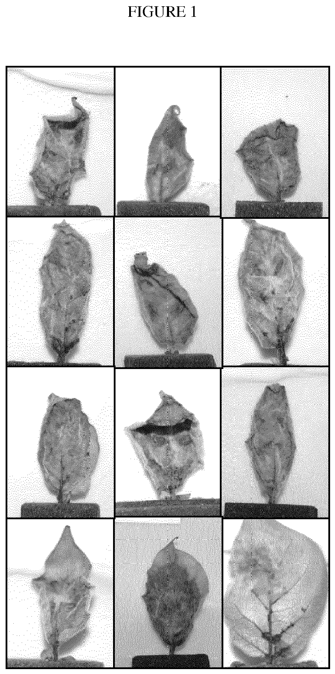

**Figure 1** shows a detached leaf assay of control potato plants after infection with *Phytophthora infestans,* wherein all leaves are infected by *Phytophthora infestans.*

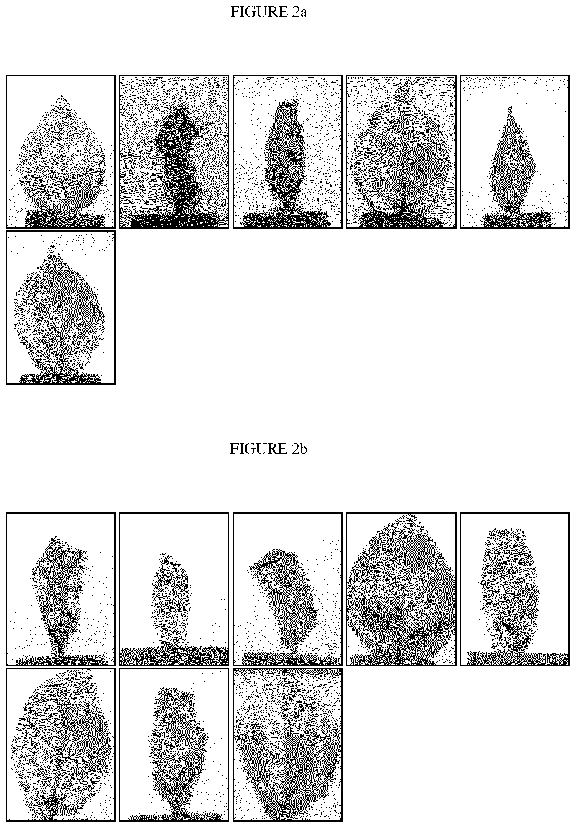

**Figure 2** shows a detached leaf assay of SEQ ID NOS. 7 & 8 silenced potato plants after infection with *Phytophthora infestans,* wherein each leaf is from an independent plant. Figure 2a shows leaves from plants silenced with a middle construct, silencing both SEQ ID NOS. 7&8. Figure 2b shows leaves from chimeric silenced plants.

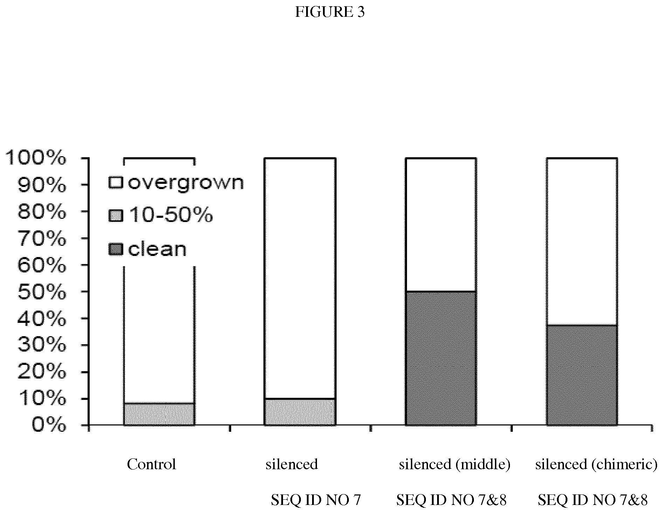

**Figure 3** shows the percentage of plants which are infected by *Phytophthora infestans,* wherein the first bar shows a control group (about 10% is partially)infected, the second bar shows plants of which only SEQ ID NO 7 is silenced (about 10% partially infected), the third bar shows plants of which both SEQ ID NO 7 and 8 is silenced in the middle part of the respective sequences (about 50% clean), the fourth bar shows plants of which both SEQ ID NO 7 and 8 is silenced at the 5'end (about 40% clean).

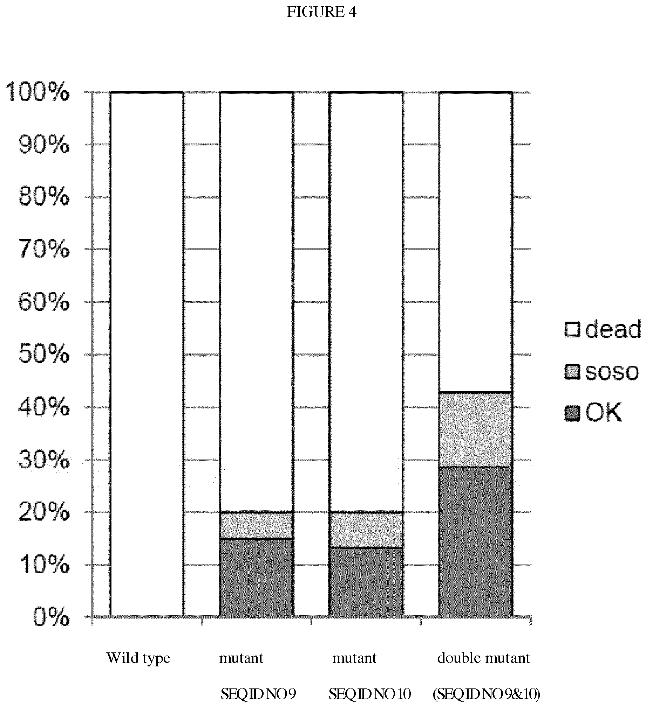

**Figure 4** shows the percentages of living petunia plants after inoculation with *Phytophthora nicotianae,* wherein the first bar shows wild type control plant (0% living), the second bar shows SEQ ID NO 9 mutants (20% living plants), the third bar shows SEQ ID NO 10 mutants (20% living plants) and the fourth bar shows double mutants, i.e. both SEQ ID NO 9 and 10 (45% living plants).

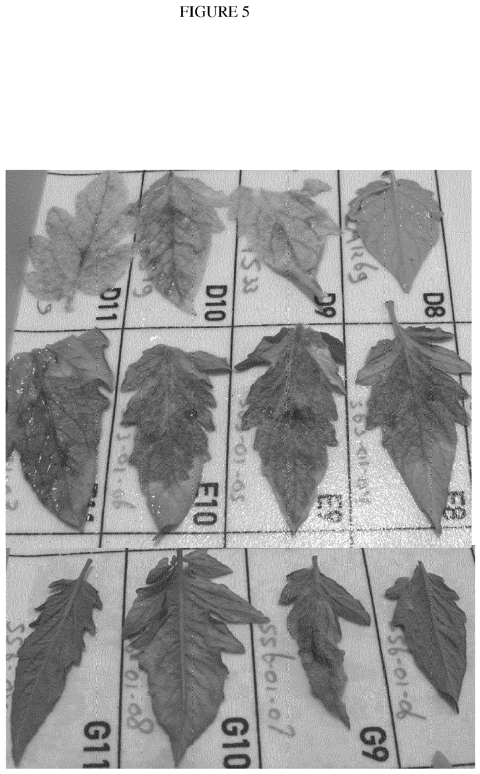

**Figure 5** shows leaves of tomato plants from a *Phytophthora infestans* disease test.

### Examples

### Example 1 (potato)

### RNAi constructs targeting potato SEQ ID NOS. 7 and 8

3 different RNAi constructs were made, harboring/targeting:
1. 5' end of SEQ ID NO 7: equivalent to coding sequence -159-200 (-159 from start means in 5'utr).
2. Chimera of 5' end of SEQ ID NOS. 7 and 8: equivalent to coding sequence 4-199 + 1-204.
3. Middle part of SEQ ID NO. 7 (highly homologous to middle of SEQ ID NO 8): equivalent to coding sequence 334-743.

The fragments were amplified from genomic DNA and cloned into the pENTR-D-TOPO vector. For the chimeric construct, 2 fragments were coupled using primers with complementary overhangs, and subsequent extension and amplification to create the fused fragment. Fragments were transferred using a Gateway LR reaction to the RNAi vector pK7GWiWG2 (Karimi et al., 2002, Trends Plant Sci 7), creating an inverted repeat with hairpin structure. Because the pK7GWiWG2 vector requires Streptomycin for bacterial selection, and the Agrobacterium strain used for potato transformation (LBA4404) already carries a Streptomycin selection marker, the complete RNAi (hairpin) cassette was transferred to a different plant transformation vector, pGreen0029 (bacterial as well as plant selection marker = Kanamycin) (Hellens et al., 2000, Plant Mol Biol 42). The final constructs allow stable expression of a 35S-promoter driven hairpin RNA that forms a silencing-inducing dsRNA, after the hairpin-loop forming intron gets spliced out. At least six independent T1 transformants were maintained for each construct.

### Phytophthora infestans Assay details

Detached leaves were taken from T1 (first generation transgenics) plants, and placed in a tray with 100% RH with petioles in wet cotton-wool or Oasis. *Phytophthora infestans* (P.inf) zoospores/sporangia were harvested from P.inf cultures (rye-sucrose-agar plates), and a 10ul drop of spore suspension containing 10e3 sporangia (10e5/ml) was placed on each side of the midvein. Trays were incubated at 18C. Leaf infection rates were scored on day 11, as 1. Completely infected/overgrown, 2. Partially infected (10-50% area), and 3. Clean (<10% area).

As shown in figures 1 and 2, the double silenced (SEQ ID NO. 7 & 8) plants of figure 2a show that only 50% is infected, the double silenced (chimeric) plants of figure 2b show that only 60% is infected, whereas the control group of figure 1 shows that all plants were infected. As shown in figure 3, 40 to 50% of the both SEQ ID NO. 7 and SEQ ID NO. 8 silenced plants are clean, whereas the plants having only SEQ ID NO. 7 silenced only 10% of the plants score partially infected. Accordingly, silencing of both SEQ ID NO. 7 and 8 provides resistance to *Phytophthora infestans.*

### Example 2 (petunia)

Transposon insertion lines were identified from a collection/library (Vandenbussche et al., 2008, Plant Journal 54). 2 dTph1 transposon insertion alleles were found in SEQ ID NO 9 and 3 dTph1 transposon insertion alleles in SEQ ID NO 10. Several crosses were made to generate double mutants.

### Phytophthora nicotianae Assay details

Plants were grown in standard potting soil, individually potted, at 23C.
*P.nicotianae* spores were harvested from cultures (lima-bean-agar or V8-agar plates), and 2ml of spore suspension containing 10e4 (assay Sept) spores was dripped onto the soil with each plant. Plant collapse was monitored regularly.

As shown in figure 4, double mutants, i.e. plants having mutations in both SEQ ID NO 9 and SEQ ID NO 10 have a percentage of living plants of 45%, whereas the percentage of living plants of single mutants (mutant in SEQ ID NO. 9 or SEQ ID NO. 10) is only 20%.

### Example 3 (tomato)

Tomato plants were transformed with two constructs, either for providing over expression of both SEQ ID NO. 11 and 12, or for providing silencing of both SEQ ID NO. 11 and 12.

Tomato SEQ ID NO. 11 silencing constructs were generated using Gateway cloning of a 300 bp fragment identical to the middle part of the CDS of SEQ ID NO. 11.

### Sequence:

### Using primers:

| | |
|---|---|
| S. Lycopersicum AttB1-F | aaaaagcaggcttcttgggtgaacaaggacaaca |
| S.Lycopersicum AttB2-R | agaaagctgggtaaaacgaagccactgacatcc |

The generated ENTRY vector was Gateway cloned into the pHellsgate12 binary vector. Following Agrobacterium transformation according standard procedure for tomato. The silencing constructs were able to silence both SEQ ID NO. 11 and 12, due to similarities in the sequences.

Offspring from transformed tomato plants were subjected to a disease test by inoculation of *Phytophthora infestans* isolate US11. 7 days after inoculation the plants were visually analysed by scoring leaves on a visual scale from 1 to 9, wherein 1 means susceptible and 9 means resistant. As a control for susceptible the plants TS33, TS19 and OT9 were used. As control for resistant the known resistant wild accession LA1269 is used. Per plant 8 leaves were measured. Table below provides the average score from the 8 leaves per plant.

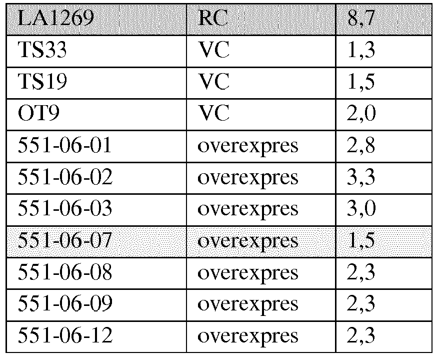

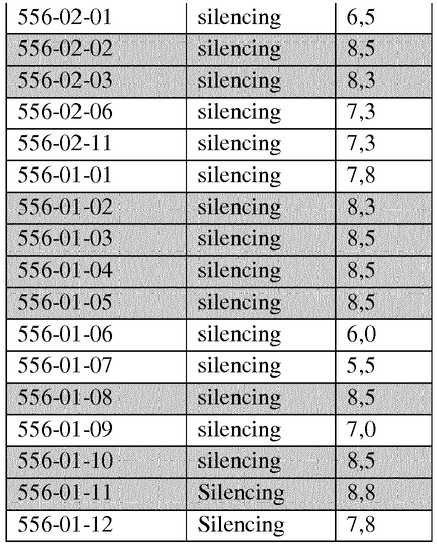

In the table is shown that the SEQ ID NO. 11 and 12overexpressing plants are susceptible for isolate US11. The silenced plant provides significant higher scores than the susceptible control LA1269.. For example plant 556-01-08 has an average score of 8.5. A sample of this plant is shown in figure 5 in box G10, and is not infected similar to resistant control plant LA1296 as shown in box D8. Accordingly, silencing of both SEQ ID NO. 11 and 12 provides resistance to *Phytophthora infestans.*

## Claims

1. Plant belonging to the *Solanaceae* family wherein said plant comprises a genetic trait providing *Phytophthora* resistance and wherein said resistance trait is encoded by a combination of at least two genes having a reduced expression of said genes as compared to said plant belonging to *Solanaceae* family being susceptible to *Phytophthora* wherein:
- said plant is potato, said *Phytophthora* resistance is resistance to *Phytophthora infestans* and said combination of at least two genes are genes encoding proteins according to SEQ ID No. 1 and SEQ ID No. 2 or proteins having at least 90% sequence identity with SEQ ID No. 1 and SEQ ID No. 2; or
- said plant is petunia, said *Phytophthora* resistance is resistance to *Phytophthora nicotianae* and said combination of at least two genes are genes encoding proteins according to SEQ ID No. 3 and SEQ ID No. 4 or proteins having at least 90% sequence identity with SEQ ID No. 3 and SEQ ID No. 4; or
- said plant is tomato, said *Phytophthora* resistance is resistance to *Phytophthora infestans* and said combination of at least two genes are genes encoding proteins according to SEQ ID No. 5 and SEQ ID No. 6 or proteins having at least 90% sequence identity with SEQ ID No. 5 and SEQ ID No. 6.

## Patentansprüche

1. Zur Familie der *Solanaceae* gehörige Pflanze, wobei diese Pflanze ein genetisches Merkmal aufweist, das *Phytophthora-Resistenz* verleiht, und wobei dieses Resistenzmerkmal durch eine Kombination von wenigstens zwei Genen codiert ist, die im Vergleich zu der zur Familie der *Solanaceae* gehörigen Pflanze, die für *Phytophthora* anfällig ist, eine reduzierte Expression dieser Gene haben, wobei
- die Pflanze eine Kartoffel ist, die *Phytophthora-Resistenz* eine Resitenz gegen *Phytophthora infestans* ist und die Kombination von wenigstens zwei Genen Gene sind, die Proteine gemäß der SEQ ID Nr. 1 und der SEQ ID Nr. 2 codieren oder Proteine mit einer Sequenzidentität mit der SEQ ID Nr. 1 und der SEQ ID Nr. 2 von wenigstens 90%; oder
- die Pflanze eine Petunie ist, die *Phytophthora-Resistenz* eine Resistenz gegen *Phytophthora nicotianae* ist und die Kombination von wenigstens zwei Genen Gene sind, die Proteine gemäß der SEQ ID Nr. 3 und der SEQ ID Nr. 4 codieren oder Proteine mit einer Sequenzidentität mit der SEQ ID Nr. 3 und der SEQ ID Nr. 4 von wenigstens 90%; oder
- die Pflanze eine Tomate ist, die *Phytophthora-Resistenz* eine Resistenz gegen *Phytophthora infestans* ist und die Kombination von wenigstens zwei Genen Gene sind, die Proteine gemäß der SEQ ID Nr. 5 und der SEQ ID Nr. 6 codieren oder Proteine mit einer Sequenzidentität mit der SEQ ID Nr. 5 und der SEQ ID Nr. 6 von wenigstens 90%.

## Revendications

1. Plante appartenant à la famille *Solanaceae* dans laquelle ladite plante comprend un trait génétique fournissant une résistance à *Phytophthora* et dans laquelle ledit trait de résistance est codé par une combinaison d'au moins deux gènes ayant une expression réduite desdits gènes en comparaison à ladite plante appartenant à la famille *Solanaceae* étant sensible à *Phytophthora,* dans laquelle :
- ladite plante est la pomme de terre, ladite résistance à *Phytophthora* est une résistance à *Phytophthora infestans* et ladite combinaison des au moins deux gènes comporte des gènes codant pour des protéines selon SEQ ID N° 1 et SEQ ID N° 2 ou des protéines ayant une identité de séquence d'au moins 90 % avec SEQ ID N° 1 et SEQ ID N° 2; ou
- ladite plante est le pétunia, ladite résistance à *Phytophthora* est une résistance à *Phytophthora nicotianae* et ladite combinaison d'au moins deux gènes comporte des gènes codant pour des protéines selon SEQ ID N° 3 et SEQ ID N° 4 ou des protéines ayant une identité de séquence d'au moins 90 % avec SEQ ID N° 3 et SEQ ID N° 4; ou
- ladite plante est la tomate, ladite résistance à *Phytophthora* est une résistance à *Phytophthora infestans* et ladite combinaison d'au moins deux gènes comporte des gènes codant pour des protéines selon SEQ ID N° 5 et SEQ ID N° 6 ou des protéines ayant une identité de séquence d'au moins 90 % avec SEQ ID N° 5 et SEQ ID N° 6.


### SEQUENCE LISTING

<110> Enza Zaden Beheer B.V.
<120> PHYTOPHTHORA REASISTANT PLANTS BELONGING TO THE SOLANACEAE FAMILY
<130> 4/2PL75/50
<160> 12
<170> BiSSAP 1.2
<210> 1
   <211> 337
   <212> PRT
   <213> Solanum tuberosum
<400> 1
   Met Glu Thr Lys Val Ile Ser Ser Gly Ile His His Ser Thr Leu Pro
   1 5 10 15
   Gln Ser Tyr Ile Arg Pro Glu Ser Asp Arg Pro Arg Leu Ser Asp Val
   20 25 30
   Val Asp Cys Glu Asn Val Pro Ile Ile Asp Leu Gly Cys Gly Asp Gln
   35 40 45
   Ala Gln Ile Ile Arg Leu Ile Gly Glu Ala Cys Gln Thr Tyr Gly Phe
   50 55 60
   Phe Gln Val Ile Asn His Gly Val Pro Lys Glu Val Val Glu Lys Met
   65 70 75 80
   Leu Gly Val Ala Gly Glu Phe Phe Asn Leu Pro Val Glu Glu Lys Leu
   85 90 95
   Lys Leu Tyr Ser Asp Asp Pro Ser Lys Thr Met Arg Leu Ser Thr Ser
   100 105 110
   Phe Asn Val Lys Lys Glu Thr Val His Asn Trp Arg Asp Tyr Leu Arg
   115 120 125
   Leu His Cys His Pro Leu Glu Lys Tyr Ala Pro Glu Trp Pro Ser Asn
   130 135 140
   Pro Ser Ser Phe Arg Asp Ile Val Ser Arg Tyr Cys Thr Glu Val Arg
   145 150 155 160
   Gln Leu Gly Phe Arg Leu Glu Glu Ala Ile Ala Glu Ser Leu Gly Leu
   165 170 175
   Glu Lys Glu Cys Ile Lys Asp Val Leu Gly Glu Gln Gly Gln His Met
   180 185 190
   Ala Ile Asn Phe Tyr Pro Pro Cys Pro Gln Pro Glu Leu Thr Tyr Gly
   195 200 205
   Leu Pro Ala His Thr Asp Pro Asn Ser Leu Thr Ile Leu Leu Gln Asp
   210 215 220
   Leu Gln Val Ser Gly Leu Gln Val Leu Lys Asp Gly Lys Trp Leu Ala
   225 230 235 240
   Val Lys Pro Gln Pro Asp Ala Phe Val Ile Asn Leu Gly Asp Gln Leu
   245 250 255
   Gln Ala Val Ser Asn Gly Lys Tyr Lys Ser Val Trp His Arg Ala Ile
   260 265 270
   Val Asn Ser Asp Gln Ala Arg Met Ser Val Ala Ser Phe Leu Cys Pro
   275 280 285
   Cys Asp Ser Ala Lys Ile Ser Ala Pro Lys Leu Leu Thr Glu Asp Gly
   290 295 300
   Ser Pro Val Ile Tyr Gln Asp Phe Thr Tyr Ala Glu Tyr Tyr Lys Lys
   305 310 315 320
   Phe Trp Ser Arg Asn Leu Asp Gln Glu His Cys Leu Glu Leu Phe Lys
   325 330 335
   Asn
<210> 2
   <211> 342
   <212> **PRT**
   <213> Solanum tuberosum
<400> 2
   Met Glu Thr Thr Ser Val Leu Ser Gly Gly Phe Asn His Ser Thr Leu
   1               5                   10                  15
   Pro Glu Ser Tyr Val Arg Pro Glu Ser Gln Arg Pro Arg Met Ser Glu
               20                  25                  30
   Val Val Asp Arg Asp Asp Leu Val Pro Val Ile Asp Met Ser Cys Thr
           35                  40                  45
   Asp Arg Asn Val Ile Val His Gln Ile Gly Glu Ala Cys Arg Leu Tyr
       50                  55                  60
   Gly Phe Phe Gln Val Ile Asn His Gly Val Ser Lys Lys Val Met Asp
   65                  70                  75                  80
   Glu Met Leu Gly Val Ala His Glu Phe Phe Lys Leu Pro Val Glu Glu
                   85                  90                  95
   Lys Met Lys Leu Tyr Ser Asp Asp Pro Ser Lys Thr Met Arg Leu Ser
               100                 105                 110
   Thr Ser Phe Asn Val Lys Lys Glu Thr Val His Asn Trp Arg Asp Tyr
           115                 120                 125
   Leu Arg Leu His Cys Tyr Pro Leu Asp Lys Tyr Ala Pro Glu Trp Pro
       130                 135                 140
   Ser Asn Pro Pro Ser Phe Arg Glu Ile Val Ser Lys Tyr Cys Met Glu
   145                 150                 155                 160
   Val Arg Gln Val Gly Tyr Arg Leu Glu Glu Ala Ile Ser Glu Ser Leu
                   165                 170                 175
   Gly Leu Glu Lys Asp Cys Ile Lys Asn Val Leu Gly Glu Gln Gly Gln
               180                 185                 190
   His Met Ala Ile Asn Phe Tyr Pro Pro Cys Pro Gln Pro Glu Leu Thr
           195                 200                 205
   Tyr Gly Leu Pro Ala His Thr Asp Pro Asn Ala Ile Thr Ile Leu Leu
       210                 215                 220
   Gln Asp Leu Gln Val Ala Gly Leu Gln Val Leu Lys Asp Gly Glu Trp
   225                 230                 235                 240
   Leu Ser Ile Lys Pro Gln Pro Asp Ala Phe Val Ile Asn Leu Gly Asp
                   245                 250                 255
   Gln Leu Glu Ala Leu Ser Asn Gly Lys Tyr Lys Ser Ile Trp His Arg
               260                 265                 270
   Ala Ile Val Asn Ser Asp Lys Ala Arg Met Ser Val Ala Ser Phe Leu
           275                 280                 285
   Cys Pro Asn Asp Cys Ser Ile Ile Ser Ala Pro Lys Thr Leu Ile Glu
       290                 295                 300
   Asp Gly Ser Ser Ala Ile Tyr Arg Asp Phe Thr Tyr Thr Glu Tyr Tyr
   305                 310                 315                 320
   Asp Lys Phe Trp Ser Arg Asn Leu Asp Gln Glu Tyr Cys Leu Glu Leu
                   325                 330                 335
   Phe Lys Asn Asp Gly Thr
               340
<210> 3
   <211> 340
   <212> PRT
   <213> Petunia
<400> 3
   Met Glu Ser Asn Val Ile Ser Ser Gly Thr Lys Tyr Thr Asn Leu Pro
   1               5                   10                  15
   Lys Ser Tyr Val Arg Pro Glu Ser Gln Arg Pro Arg Leu Ser Glu Val
                   20                  25                  30
   Asp Asp Cys Gln Asp Asn Ile Pro Val Ile Asp Leu Cys Cys Arg Asp
           35                  40                  45
   Asn Asn Val Ile Ile Gln Gln Ile Glu Glu Ala Cys Arg Leu Tyr Gly
       50                  55                  60
   Phe Phe Gln Val Ile Asn His Gly Val Pro Lys Lys Leu Ile Glu Glu
   65                  70                  75                  80
   Met Leu Gly Val Ala His Glu Phe Phe Lys Leu Pro Val Glu Glu Lys
                   85                  90                  95
   Met Lys Leu Tyr Ser Asp Asp Pro Ser Lys Thr Met Arg Leu Ser Thr
               100                 105                 110
   Ser Phe Asn Val Lys Lys Glu Thr Val His Asn Trp Arg Asp Tyr Leu
           115                 120                 125
   Arg Leu His Cys Tyr Pro Leu Glu Lys Tyr Ala Pro Glu Trp Pro Ser
       130                 135                 140
   Thr Pro Ser Ser Phe Arg Glu Ile Val Ser Arg Tyr Cys Ile Glu Val
   145                 150                 155                 160
   Arg Gln Leu Gly Tyr Arg Leu Gln Glu Ala Ile Ser Glu Ser Leu Gly
                   165                 170                 175
   Leu Glu Lys Asp Cys Ile Lys Asn Ile Leu Gly Glu Gln Gly Gln His
               180                 185                 190
   Met Ala Val Asn Tyr Tyr Pro Pro Cys Pro Glu Pro Glu Leu Thr Tyr
           195                 200                 205
   Gly Leu Pro Ala His Thr Asp Pro Asn Ala Leu Thr Ile Leu Leu Gln
       210                 215                 220
   Asp Leu Gln Val Ala Gly Leu Gln Val Leu Lys Asp Gly Lys Trp Leu
   225                 230                 235                 240
   Ser Val Lys Pro Arg Ala Asn Ala Phe Val Ile Asn Leu Gly Asp Gln
                   245                 250                 255
   Leu Gln Ala Leu Ser Asn Gly Lys Tyr Arg Ser Val Trp His Arg Ala
               260                 265                 270
   Ile Val Asn Ser Asp Lys Pro Arg Leu Ser Val Ala Ser Phe Leu Cys
           275                 280                 285
   Pro Ser Asp Cys Ala Ile Ile Ser Ala Pro Lys Thr Leu Thr Glu Asp
       290                 295                 300
   Gly Ser Pro Thr Ile Tyr Arg Asp Phe Thr Tyr Pro Glu Tyr Tyr Lys
   305                 310                 315                 320
   Lys Phe Trp Ser Arg Asn Leu Asp Gln Glu His Cys Met Glu Leu Phe
                   325                 330                 335
   Lys Lys Gly Ser
               340
<210> 4
   <211> 337
   <212> PRT
   <213> Petunia
<400> 4
   Met Glu Thr Lys Val Leu Ser Ser Gly Ile Arg His Ser Thr Leu Pro
   1               5                   10                  15
   Gln Asn Tyr Val Arg Pro Lys Ser Asp Arg Pro Arg Leu Ser Glu Val
               20                  25                  30
   Ala Asn Cys Glu Asn Val Pro Val Ile Asp Leu Gly Cys Ala Asp Arg
           35                  40                  45
   Thr Leu Ile Ile His Gln Ile Ser Glu Ala Cys Arg Leu Tyr Gly Phe
       50                  55                  60
   Phe Gln Val Ile Asn His Gly Val Pro Lys Lys Ile Val Glu Glu Met
   65                  70                  75                  80
   Leu Glu Ile Ala Gly Glu Phe Phe Arg Leu Pro Val Glu Glu Lys Leu
                   85                  90                  95
   Lys Leu Tyr Ser Asp Asp Pro Ser Lys Thr Met Arg Leu Ser Thr Ser
               100                 105                 110
   Phe Asn Val Lys Lys Glu Thr Val His Asn Trp Arg Asp Tyr Leu Arg
           115                 120                 125
   Leu His Cys Tyr Pro Leu Glu Lys Tyr Ala Pro Glu Trp Pro Ser Asn
       130                 135                 140
   Pro Ser Ser Phe Arg Glu Ile Val Ser Arg Tyr Cys Thr Glu Val Arg
   145                 150                 155                 160
   Gln Leu Gly Phe Arg Leu Gln Glu Ala Ile Ala Glu Ser Leu Gly Leu
                   165                 170                 175
   Glu Lys Glu Cys Ile Lys Asp Val Leu Gly Glu Gln Gly Gln His Met
               180                 185                 190
   Ala Ile Asn Phe Tyr Pro Pro Cys Pro Glu Pro Glu Leu Thr Tyr Gly
           195                 200                 205
   Leu Pro Ala His Thr Asp Pro Asn Ala Leu Thr Ile Leu Leu Gln Asp
       210                 215                 220
   Leu Gln Val Ala Gly Leu Gln Val Leu Lys Asp Gly Lys Trp Leu Ala
   225                 230                 235                 240
   Val Lys Pro Gln Pro Asp Ala Phe Val Val Asn Leu Gly Asp Gln Leu
                   245                 250                 255
   Gln Ala Val Ser Asn Gly Arg Tyr Lys Ser Val Trp His Arg Ala Val
               260                 265                 270
   Val Asn Thr Glu Asn Ala Arg Met Ser Val Ala Ser Phe Leu Cys Pro
           275                 280                 285
   Cys Asp Ser Ala Lys Ile Ser Ala Pro Lys Leu Leu Thr Asp Asp Gly
       290                 295                 300
   Ser Pro Ile Ile Tyr Arg Asp Phe Thr Tyr Ala Glu Tyr Tyr Lys Lys
   305                 310                 315                 320
   Phe Trp Ser Arg Asn Leu Asp Gln Glu His Cys Leu Glu Leu Phe Lys
                   325                 330                 335
   Asn
<210> 5
   <211> 337
   <212> PRT
   <213> Solanum lycopersicum
<400> 5
   Met Glu Thr Lys Val Ile Ser Ser Gly Ile Asn His Ser Thr Leu Pro
   1               5                   10                  15
   Gln Ser Tyr Ile Arg Pro Glu Ser Asp Arg Pro Arg Leu Ser Glu Val
               20                  25                  30
   Val Asp Cys Glu Asn Val Pro Ile Ile Asp Leu Ser Cys Gly Asp Gln
           35                  40                  45
   Ala Gln Ile Ile Arg Gln Ile Gly Glu Ala Cys Gln Thr Tyr Gly Phe
       50                  55                  60
   Phe Gln Val Ile Asn His Gly Val Pro Lys Glu Val Val Glu Lys Met
   65                  70                  75                  80
   Leu Gly Val Ala Gly Glu Phe Phe Asn Leu Pro Val Glu Glu Lys Leu
                   85                  90                  95
   Lys Leu Tyr Ser Asp Asp Pro Ser Lys Thr Met Arg Leu Ser Thr Ser
               100                 105                 110
   Phe Asn Val Lys Lys Glu Thr Val His Asn Trp Arg Asp Tyr Leu Arg
           115                 120                 125
   Leu His Cys Tyr Pro Leu Glu Lys Tyr Ala Pro Glu Trp Pro Ser Asn
       130                 135                 140
   Pro Ser Ser Phe Arg Glu Ile Val Ser Arg Tyr Cys Arg Glu Ile Arg
   145                 150                 155                 160
   Gln Leu Gly Phe Arg Leu Glu Glu Ala Ile Ala Glu Ser Leu Gly Leu
                   165                 170                 175
   Asp Lys Glu Cys Ile Lys Asp Val Leu Gly Glu Gln Gly Gln His Met
               180                 185                 190
   Ala Ile Asn Tyr Tyr Pro Pro Cys Pro Gln Pro Glu Leu Thr Tyr Gly
           195                 200                 205
   Leu Pro Ala His Thr Asp Pro Asn Ser Leu Thr Ile Leu Leu Gln Asp
       210                 215                 220
   Leu Gln Val Ala Gly Leu Gln Val Leu Lys Asp Gly Lys Trp Leu Ala
   225                 230                 235                 240
   Val Lys Pro Gln Pro Asp Ala Phe Val Ile Asn Leu Gly Asp Gln Leu
                   245                 250                 255
   Gln Ala Val Ser Asn Gly Lys Tyr Arg Ser Val Trp His Arg Ala Ile
               260                 265                 270
   Val Asn Ser Asp Gln Ala Arg Met Ser Val Ala Ser Phe Leu Cys Pro
           275                 280                 285
   Cys Asp Ser Ala Lys Ile Ser Ala Pro Lys Leu Leu Thr Glu Asp Gly
       290                 295                 300
   Ser Pro Val Ile Tyr Gln Asp Phe Thr Tyr Ala Glu Tyr Tyr Asn Lys
   305                 310                 315                 320
   Phe Trp Ser Arg Asn Leu Asp Gln Gln His Cys Leu Glu Leu Phe Lys
                   325                 330                 335
   Asn
<210> 6
   <211> 320
   <212> PRT
   <213> Solanum lycopersicum
<400> 6
   Met Met Thr Thr Thr Ser Val Leu Ser Ser Gly Phe Asn His Ser Thr
   1               5                   10                  15
   Leu Pro Gln Ser Tyr Val Arg Pro Glu Ser Gln Arg Pro Cys Met Ser
               20                  25                  30
   Glu Val Val Asp Ser Asp Asp Leu Val Pro Val Ile Asp Met Ser Cys
           35                  40                  45
   Thr Asp Arg Asn Val Ile Val His Gln Ile Gly Glu Ala Cys Arg Leu
       50                  55                  60
   Tyr Gly Phe Phe Gln Val Ile Asn His Gly Val Ser Lys Lys Ala Met
   65                  70                  75                  80
   Asp Glu Met Leu Gly Thr Met Arg Leu Ser Thr Ser Phe Asn Val Lys
                   85                  90                  95
   Lys Glu Thr Val His Asn Trp Arg Asp Tyr Leu Arg Leu His Cys Tyr
               100                 105                 110
   Pro Leu Asp Lys Tyr Ala Pro Glu Trp Pro Ser Asn Pro Pro Ser Phe
           115                 120                 125
   Arg Glu Ile Val Ser Lys Tyr Cys Met Glu Val Arg Glu Leu Gly Tyr
       130                 135                 140
   Arg Leu Glu Glu Ala Ile Ser Glu Ser Leu Gly Leu Glu Lys Asp Cys
   145                 150                 155                 160
   Ile Lys Asn Val Leu Gly Glu Gln Gly Gln His Met Ala Ile Asn Phe
                   165                 170                 175
   Tyr Pro Gln Cys Pro Gln Pro Glu Leu Thr Tyr Gly Leu Pro Ala His
               180                 185                 190
   Thr Asp Pro Asn Ala Ile Thr Ile Leu Leu Gln Asp Leu Gln Val Ala
           195                 200                 205
   Gly Leu Gln Val Leu Lys Asp Gly Lys Trp Leu Ser Ile Lys Pro Gln
       210                 215                 220
   Pro Asn Ala Phe Val Ile Asn Leu Gly Asp Gln Leu Glu Ala Leu Ser
   225                 230                 235                 240
   Asn Gly Lys Tyr Lys Ser Ile Trp His Arg Ala Ile Val Asn Ser Asp
                   245                 250                 255
   Lys Ala Arg Met Ser Val Ala Ser Phe Leu Cys Pro Asn Asp Cys Ser
               260                 265                 270
   Ile Ile Ser Ala Pro Lys Thr Leu Thr Glu Asp Gly Ser Ser Ala Ile
           275                 280                 285
   Tyr Arg Asp Phe Thr Tyr Ala Glu Tyr Tyr Glu Lys Phe Trp Ser Arg
       290                 295                 300
   Asn Leu Asp Gln Glu Tyr Cys Leu Glu Leu Phe Lys Asn Asp Gly Thr
   305                 310                 315                 320
<210> 7
   <211> 1014
   <212> DNA
   <213> Solanum tuberosum
<220>
   <221> source
   <222> 1..1014
   <223> /mol_type="unassigned DNA" /organism="Solanum tuberosum"
<400> 7
   atggaaacga aagttatttc cagcggaatc caccactcta ctctccctca aagttacatc      60
   cgacccgaat ccgataggcc acgtctatcg gatgtggtcg attgcgaaaa tgttccaata     120
   attgacttag gttgcggaga ccaagctcaa ataatccgtc taattggaga agcttgtcaa     180
   acttatggtt tctttcaggt aattaatcat ggtgtaccaa aggaagttgt agagaaaatg     240
   ctaggggtag ctggggaatt tttcaatcta ccagtagaag agaagctaaa attgtattca     300
   gatgatcctt caaagaccat gagattatct actagtttta atgttaaaaa ggagacagtt     360
   cataattgga gagattatct cagacttcat tgtcatcctc tggagaaata tgctcctgaa     420
   tggccttcta atccatcgtc tttcagggat atcgtgagca gatattgcac ggaagttcga     480
   caactcggat ttagattgga ggaagccata gcagagagcc tgggcttaga gaaagagtgt     540
   attaaagatg tattgggaga acaaggccaa catatggcta tcaattttta tcctccttgt     600
   ccacaaccag aactcacata tgggcttccg gcccatactg atccaaattc acttacaatt     660
   cttcttcaag acttgcaagt ttctggtctt caagttctta aagatggtaa atggttggct     720
   gtcaaacctc aaccagatgc ctttgtcatt aatcttggtg atcaattgca ggcagtaagt     780
   aacggtaagt acaaaagtgt atggcatcga gctattgtga attcagatca agctaggatg     840
   tcagtggctt cgttcctatg tccgtgcgat agcgcgaaaa tcagtgctcc aaaactcctg     900
   acagaagatg gatctccagt catttatcag gacttcacgt atgctgagta ttacaagaag     960
   ttctggagca ggaatttgga ccaggaacat tgtttggaac ttttcaagaa ttaa          1014
<210> 8
   <211> 1029
   <212> DNA
   <213> Solanum tuberosum
<220>
   <221> source
   <222> 1..1029
   <223> /mol_type="unassigned DNA" /organism="Solanum tuberosum"
<400> 8
   atggaaacaa caagtgttct ttccggtgga ttcaaccact caaccctccc tgaatcttac      60
   gttcgacctg aatcccaaag accccgcatg tctgaagttg ttgatcgtga tgatcttgtt     120
   ccagttatcg atatgtcttg tactgatagg aacgttatcg ttcatcaaat tggcgaagct     180
   tgtcgccttt atgggttttt ccaggtgata aatcacggtg tatcaaagaa ggttatggat     240
   gaaatgttgg gggtagctca tgaatttttt aagcttccag tggaagaaaa gatgaaattg     300
   tactcagatg atccatcaaa gactatgaga ttatcaacta gttttaatgt taagaaggaa     360
   actgttcata attggagaga ttatcttagg ctacactgtt atcctttgga caaatatgcc     420
   cctgaatggc cttctaatcc tccttctttc agggaaatag tgagcaaata ttgcatggaa     480
   gttagacaag ttggatatag attagaagaa gcaatatcag agagcctagg gctcgagaaa     540
   gattgtatta aaaatgtgtt gggtgaacaa ggacaacata tggctatcaa tttttatcct     600
   ccatgtccac aacctgaact aacttatggg ttaccagccc atacagatcc aaatgcaatt     660
   acaattcttc ttcaagattt gcaagtggct ggccttcaag ttcttaagga tggagaatgg     720
   ttatctatta aacctcaacc tgatgccttt gtcatcaatc ttggtgatca attggaggca     780
   ttgagtaatg gaaagtataa aagtatatgg catagagcta ttgtaaattc agataaagca     840
   aggatgtctg tggcttcttt cctctgtccc aatgattgtt ccattatcag tgctccaaaa     900
   accttaattg aagatggatc ttcagccatt tatcgagatt tcacttatac tgaatattat     960
   gacaaatttt ggagcaggaa tttagaccag gaatattgtt tagaactttt caagaacgat    1020
   ggaacctag                                                            1029
<210> 9
   <211> 1014
   <212> DNA
   <213> Petunia
<220>
   <221> source
   <222> 1..1014
   <223> /mol_type="unassigned DNA" /organism="Petunia"
<400> 9
   atggaaacaa aagttctttc aagtggaatc cgtcattcta ccctccctca aaattatgtc      60
   cgacccaaat ccgataggcc acgtctttca gaagtggcca attgtgaaaa cgttccagtt     120
   attgacttgg gttgtgctga cagaactctc ataattcatc aaattagcga agcctgtcgt     180
   ctttatggtt ttttccaggt aataaaccat ggtgtaccaa aaaaaatagt tgaggaaatg     240
   ctagagatag ctggggagtt ttttaggcta ccagttgaag agaagcttaa gttgtattca     300
   gatgaccctt caaagaccat gagattatca actagtttta atgtaaagaa ggagacggtg     360
   cacaattgga gagattatct cagacttcat tgttatcctc tggagaaata tgctcctgaa      420
   tggccttcaa atccttcatc tttcagggaa atcgtgagca gatattgcac ggaagttcga      480
   caacttggat tcagattgca agaagccata gcagaaagct taggcttaga gaaagagtgt      540
   ataaaggatg tgttaggtga acaaggtcaa catatggcta taaactttta tcctccatgc      600
   ccagaaccag aactcactta cgggctgcca gcccataccg atccaaatgc tcttacaatt      660
   cttcttcaag acttgcaagt agctggtctc caagttctta aagatggcaa atggttggct      720
   gtcaaacctc agcccgatgc ctttgttgtt aatctcggtg atcaactgca ggcagtgagt      780
   aacggaaggt acaaaagcgt atggcatcga gctgttgtaa atacagaaaa tgccaggatg      840
   tctgtggctt cgttcttatg tccctgtgat agtgcaaaaa tcagtgctcc aaaactcctc      900
   actgatgatg gatctccaat aatttatcgg gacttcacgt atgcagagta ttacaagaag      960
   ttctggagca ggaatttgga ccaagaacat tgtttggaac ttttcaagaa ttaa           1014
<210> 10
   <211> 1023
   <212> DNA
   <213> Petunia
<220>
   <221> source
   <222> 1..1023
   <223> /mol_type="unassigned DNA" /organism="Petunia"
<400> 10
   atggaatcta atgttatttc cagcggaacc aaatacacaa acctccctaa aagttatgtt      60
   cgcccagaat cccaacgacc tcggttatct gaagtagacg attgccaaga taatattcca     120
   gttattgatt tgtgttgcag agacaataac gttatcattc aacaaattga agaagcttgt     180
   cgtctttatg gcttttttca ggtaataaac catggtgtac caaagaaact aatagaggaa      240
   atgctagggg tagctcatga gtttttcaag ctaccagtgg aagagaagat gaagttgtac     300
   tcagatgatc catcaaagac catgagatta tcaacaagtt ttaatgtgaa gaaggaaact     360
   gttcataatt ggagagacta tcttagattg cactgctatc ctttggagaa atatgcccct     420
   gaatggcctt ctactccctc ttctttcagg gaaatcgtta gcagatattg catagaagtt     480
   cgacaacttg gatatagatt acaagaagca atatcagaga gcttaggcct agagaaagat     540
   tgtataaaaa atatattggg tgaacaaggt caacatatgg ctgttaatta ttaccctcca     600
   tgtccagaac cagaactaac ttatggtttg ccagcccata ctgatcctaa tgcccttact     660
   atacttcttc aagacttgca agtagcaggt cttcaagttc tcaaggatgg taaatggtta     720
   tctgtgaaac ctcgggccaa tgcctttgtc atcaatcttg gtgatcaatt gcaggcgctg     780
   agtaatggaa aatatagaag tgtatggcac agagctatag taaattcaga caaaccaagg     840
   ctgtcagtgg cttctttctt gtgtcctagt gattgtgcga taatcagtgc tccaaaaacc     900
   ttaactgaag atgggtctcc aaccatttat cgggatttca cgtatccaga atattacaag     960
   aaattttgga gcagaaattt agatcaagaa cactgtatgg aacttttcaa gaaaggaagc    1020
   tag                                                                  1023
<210> 11
   <211> 1014
   <212> DNA
   <213> Solanum lycopersicum
<220>
   <221> source
   <222> 1..1014
   <223> /mol_type="unassigned DNA" /organism="Solanum lycopersicum"
<400> 11
   atggaaacca aagttatttc tagcggaatc aaccactcta ctcttcctca aagttacatc       60
   cgacccgaat ccgatagacc acgtctatcg gaagtggtcg attgtgaaaa tgttccaata      120
   attgacttaa gttgcggaga tcaagctcaa ataattcgtc aaattggaga agcttgtcaa      180
   acttatggtt tctttcaggt aattaatcat ggtgtaccaa aggaagttgt agagaaaatg      240
   ctaggggtag ctggggaatt tttcaattta ccagtagaag agaaactaaa attatattca      300
   gatgatcctt caaagaccat gagattatca acaagtttta atgttaaaaa ggagacagtt      360
   cataattgga gagattatct cagacttcat tgttatcctc tagagaagta tgctcctgaa      420
   tggccttcta atccatcatc tttcagggaa atcgtgagca gatattgcag ggaaattcgt      480
   caactcggat ttagattaga agaagccata gcagaaagcc tggggttaga taaagagtgt      540
   ataaaagatg tattgggtga acaaggacaa catatggcta tcaattatta tcctccttgt      600
   ccacaaccag aacttactta tgggcttccg gcccatactg atccaaattc acttacaatt      660
   cttcttcaag acttgcaagt tgcgggtctt caagttctta aagatggcaa atggttagct      720
   gtaaaacctc aacctgacgc ctttgtcatt aatcttgggg atcaattgca ggcagtaagt      780
   aacggtaagt acagaagtgt atggcatcga gctattgtga attcagatca agctaggatg      840
   tcagtggctt cgtttctatg tccgtgtgat agcgcgaaaa tcagtgcacc aaagctgctg      900
   acagaagatg gatctccagt gatttatcaa gactttacgt atgctgagta ttacaacaag      960
   ttctggagca ggaatttgga ccagcaacat tgtttggaac ttttcaagaa ctaa           1014
<210> 12
   <211> 963
   <212> DNA
   <213> Solanum lycopersicum
<220>
   <221> source
   <222> 1..963
   <223> /mol_type="unassigned DNA" /organism="Solanum lycopersicum"
<400> 12
   atgatgacaa caacaagtgt tctttctagt ggattcaacc actcaaccct ccctcaatct      60
   tacgttcgac ctgaatctca aagaccttgc atgtctgaag ttgttgatag cgacgatctt     120
   gtcccagtca ttgatatgtc ttgtactgat aggaacgtta tcgttcatca aatcggtgaa     180
   gcttgtcgtc tttatgggtt ttttcaggtg ataaatcacg gtgtgtcgaa gaaggcgatg     240
   gatgaaatgt tagggactat gagattatca actagtttta atgttaagaa ggaaactgtt     300
   cataattgga gagattatct taggctacat tgttatcctt tggacaaata tgcccctgaa     360
   tggccttcta atcctccttc tttcagggaa atagtaagca aatattgcat ggaagttaga     420
   gagcttggat atagattgga agaagcaata tcagagagct tagggcttga gaaggattgt     480
   ataaaaaatg tgttaggtga acaaggacaa catatggcta tcaattttta tcctcagtgt     540
   ccacaacctg aattaactta tgggttacca gcccatacag atccaaatgc aattacaatt     600
   cttcttcaag atttgcaagt ggctggcctt caagttctta aggatggaaa atggttatct     660
   attaaacctc agcctaatgc ctttgtcatc aatcttggtg atcaattgga ggcgttgagt     720
   aatgggaagt ataaaagtat atggcataga gctattgtaa attcagacaa agcaaggatg     780
   tctgtggctt cttttctctg tcccaatgat tgttccatta tcagtgctcc aaaaacctta     840
   actgaagatg gatcttctgc aatttatcga gatttcactt atgctgaata ttatgaaaaa     900
   ttctggagca ggaatttaga tcaggaatat tgtttagaac tttttaagaa cgatggaacc     960
   tag                                                                   963